# EUROPEAN PATENT SPECIFICATION

(11) **EP 1 827 160 B1**
(45) Date of publication and mention of the grant of the patent: **24.11.2010**
(21) Application number: 05811511.4
(22) Date of filing: 24.11.2005
(51) Int. Cl.: A45D 2/48

(54) **COSMETIC PRODUCT AND METHOD OF APPLYING A MASCARA COMPOSITION**
KOSMETIKPRODUKT UND VERFAHREN ZUM AUFBRINGEN EINER MASCARAZUSAMMENSETZUNG
PRODUIT COSMETIQUE ET METHODE D'APPLICATION D'UNE FORMULE DE MASCARA

(30) Priority: 29.11.2004 WO PCT/JP2004/018091
(43) Date of publication of application: 05.09.2007
(73) Proprietor: Panasonic Electric Works Co., Ltd., Kadoma-shi Osaka (JP); The Procter and Gamble Company, Cincinnati, OH 45202 (US)
(72) Inventor: KAMADA, Kenji, Kadoma-shi, Osaka 571-8686 (JP); FUNATSU, Keiko, Kadoma-shi, Osaka 571-8686 (JP); YAMAGUCHI, Naoki, Kadoma-shi, Osaka 571-8686 (JP); HATANO, Satoru, Hyogo 6590013 (JP); LAM, Virginia, Ann, 45202 Ohio (US)
(74) Representative: Appelt, Christian W.
(86) International application number: PCT/JP2005/022047
(87) International publication number: WO 2006/057439

(56) References cited:
- EP-A- 1 466 541
- US-A1- 2001 002 228
- US-B1- 6 412 496

## Description

### TECHNICAL FIELD

The present invention relates to a cosmetic product including a mascara composition and an eyelash treatment device, whereby the mascara composition is applied to the eyelashes by fluidizing the mascara composition via heat generated by the eyelash treatment device. The product provides improved performance of a mascara composition, while also being safe for the general consumer to use. The present invention further relates to a method of applying a mascara composition to the eyelashes.

### BACKGROUND ART

Mascara products are used to enhance the beauty of a person's eyes by coating the eyelashes to primarily thicken, lengthen, color, and define the individual eyelashes. For the last 60 years, mascara products have been provided in the form of mascara applicators having an applicator brush attached to a handle, the applicator brush portion dipped in a package, such as a tube, containing a liquid to semi-solid mascara composition. Mascara compositions typically take the form of emulsions or dispersions of waxes and pigments in water or other volatile carriers. Mascara composition and delivery systems are limited by emulsion or solution chemistry and film forming technologies that are applied wet and then dried to create a film of mascara that sets and holds the eyelashes.

These mascara compositions which are liquid to semi-solid have a low viscosity profile and low yield point, such that they are inherently prone to smearing and smudging after application to the eyelashes. The solid components dispersed in the composition, such as waxes, may also be difficult to apply, as clumping and globbing may occur due to lack of film smoothness of the solid components.

From another aspect, solvents and carriers of the composition that do not evaporate in timely manner may also provide smearing and smudging after application to the eyelashes. The so-called waterproof mascaras intend to solve such problem by employing volatile hydrocarbon solvents. While such volatile hydrocarbon solvents provide wear benefits, the application and beauty benefits may be compromised. Further, the volatile hydrocarbon solvents may cause odor and safety concerns.

One solution for providing a mascara composition having improved application and improved wearability, is to provide the composition solid, wherein the composition is heated prior to application to the eyelashes, for softening, and/or smoothing the composition upon application. Such heating would also benefit in shortening the evaporation time required after application. The solid film provided on the eyelashes after application would have a much higher yield point than films made by conventional mascara compositions, thereby being less prone to smearing and smudging.

Based on the foregoing, there is a need for a mascara product which can apply a solid mascara composition to the eyelashes in a safe and effective manner. It would be further beneficial to provide the solid mascara composition in a predetermined dosage form, such that the user is easily provided with an adequate amount of composition to apply to the eyelashes.

Meanwhile, there have been proposed in the art eyelash curling devices, such as WO 99/22782 including a applicator brush and heater combination. However, the applicator brush is designed to curl the eyelashes by application of heat, while applying the mascara composition of liquid or semi-liquid condition fetched from a container. Due to the absence of the idea and structure for heating and fluidizing the solid mascara composition, the applicator brush is practically impossible to use in combination with the mascara composition proposed by the present invention. There is a need of providing a dedicated treatment device which takes the full benefit of the mascara composition to apply the fluidized composition successfully and uniformly to the eyelashes. There is a further need of providing such a treatment device which also comprises a loading means for providing a predetermined dosage form of mascara composition upon use.

EP 1 466 541 A1 relates to an eyelash treatment device comprising a grip and an applicator, wherein said applicator comprises a rod and an application portion. Said document further discloses a container holding a formulation, such as mascara. By inserting said applicator into the container, the application portion is covered with the formulation and may subsequently be applied.

US2001/0002228 A1 relates to a toothbrush with multiple pumping systems. In its handle portion, the tooth brush comprising three reservoirs containing liquids. The reservoirs are connected to the brush formed in the head portion of the tooth brush with the aid of delivery tubes.

None of the existing art provides all of the advantages and benefits of the present invention.

### DISCLOSURE OF THE INVENTION

The present cosmetic product comprises a mascara composition and an eyelash treatment device, whereby the mascara composition is applied to the eyelashes by softening the mascara composition via heat, the applied mascara composition forms a solid film on the eyelashes, which film has improved wearability. The product provides improved performance of a mascara composition, while also being safe for the general consumer to use.

The mascara composition is solid at room temperature, however is softened at elevated temperature, so it may be applied to the eyelashes. The eyelash treatment device comprises a heater for providing enough heat to the mascara composition, yet safe for application to the eyelashes, even in accidental situations where the user inadvertently touches the device with the eyelids or eyeballs. The eyelash treatment device comprises an applicator for receiving, heating (softening), and applying the mascara composition.

Once the softened mascara composition is applied to the eyelashes, the mascara composition is quickly cooled and thus solidified by the atmosphere. The mascara composition thus applied to the eyelashes provides a firm film covering the eyelashes, which is not easily softened at body temperature, and thus provides enhanced wearability.

In particular, the mascara composition of the present invention fulfills the above performances by incorporation of a solid hydrophobic component which gives a unique rhelogical profile to the composition. The rheological profile of the composition is defined in terms of a needle penetration as measured according to ASTM (American Society for Testing and Material) Test Method D5, a yield stress, and a viscosity. The mascara composition of the present invention is prepared to have the needle penetration of from about 1 to about 40, the yield stress of at least about 1500 Pa at 25 °C, and the viscosity of between about 1 mPas and about 10,000,000 mPas at 100 °C. The mascara composition with the above rheological profile can be therefore applied to the eyelashes by being softened via heat and is then solidified on the eyelashes to provide a firm film on the eyelashes.

The mascara composition may be prepared in the form of an oil mixture in which the solid hydrophobic component forms an oil phase. The composition may be alternatively in the form of a water-in-oil emulsion additionally including water and an emulsifier.

Further, the composition may be added with a pigment and/or a film forming polymer for enhanced aesthetic appeal and wearability.

The device of the present invention is specifically designed to give a structure that is configured to soften the mascara composition and to hold the softened mascara composition for applying it to the eyelashes uniformly. The device includes an applicator equipped with a heater for softening the mascara composition, and a comb arranged along the length of the applicator. The comb is arranged along the length of the applicator to be coated with the softened mascara composition. Whereby the softened mascara composition can be successfully delivered to the eyelashes from the entire length of the comb, leaving the solid mascara film on the eyelashes.

The device of the present invention is further equipped with a loading means for delivering a predetermined dosage form of mascara composition upon use. The loading means may be provided in a cap fitted over the applicator.

By providing the device with the loading means of predetermined dosage, the user may easily apply an adequate amount of composition to the eyelashes. The loading means is configured to hold a plurality of mascara pieces and to load the mascara piece one by one to the applicator for delivering the adequate dosage of the mascara composition.

The cap includes a sheath fitted over the applicator and formed with an opening communicating with the applicator. The loading means includes a carrier which has a plurality of compartments each storing the mascara piece. The carrier is movable relative to the sheath in order to make one of the compartments selectively in registration with the opening for delivering the mascara piece to the applicator through the opening. Thus, the mascara piece can be easily delivered to the applicator simply by manipulating the carrier.

Preferably, the carrier is prepared in the form of a sleeve coaxial with the sheath to be rotatable about the axis of the sheath. In this instance, the sleeve may be equipped with a plurality of gates which normally close the compartments and are movable to open the associated compartments for delivering the mascara piece.

The opening may be provided with a normally-closed valve. In this instance, the loading means is provided with a plunger which forces the mascara piece to temporarily open the flap valve for delivering the mascara piece to the applicator.

The carrier may take the form of a disc which is rotatable about an upright axis perpendicular to the axis of the sheath, and has a plurality of compartments arranged about the upright axis. Also with this arrangement, the mascara piece can be delivered simply by rotating the disc about the upright axis perpendicular to the axis of the sheath or the applicator.

The mascara piece may be retained in each of the compartments by means of a breakable seal. In this connection, the loading means is provided with a plunger which forces the mascara piece out of the breakable seal for delivering the same to the applicator. With the use of the breakable seal, the mascara composition can be kept in good condition over a long period, but is easy to be loaded to the applicator.

The compartments formed in the disc may be spaced circumferentially as well as radially such that a set of radially aligned compartments comes into registration with the opening elongated along the axis of the sheath. With this arrangement, more than one mascara piece can be delivered to axially spaced points on the applicator.

Rather than utilizing the movable carrier holding the mascara pieces, the loading means may include a feeder in the form of a screw-in piston that drives the mascara pieces linearly towards the opening with the normally-closed flap valve. The loading means is further provided with a plunger that forces the mascara piece just around the opening against the flap valve to temporarily open the valve. Thus, the mascara piece can be delivered by a combination of manipulating the feeder and the plunger.

Instead of the screw-in piston, the feeder may be in the form of a spring-biased piston so that the mascara piece can be delivered simply by pressing the plunger.

Further, the loading means may be configured to include a hopper formed at one end of the sheath away from the opening to contain the mascara pieces in the form of pellets, and a screw-conveyor extending into the compartment for feeding the pellets from the hopper to the opening. Thus, the mascara composition can be delivered simply by manipulating the screw-conveyor, and by an adequate amount or dosage by adjusting the movement of the conveyor.

These and other features, aspects, and advantages of the present invention will become evident to those skilled in the art from a reading of the present disclosure with the appended claims.

### BRIEF DESCRIPTION OF THE DRAWINGS

While the specification concludes with claims particularly pointing out and distinctly claiming the invention, it is believed that the present invention will be better understood from the following description of preferred, nonlimiting embodiments and representations taken in conjunction with the accompanying drawings in which:
FIG. 1 is an exploded perspective view of an eyelash treatment device in accordance with a preferred embodiment of the present invention;
FIG. 2 is a front view of the above device;
FIG. 3 is a top view of the applicator;
FIG. 4 is a sectional view of the applicator;
FIG. 5 is an exploded perspective view of a cap adapted in the above device for loading mascara pieces in accordance with a first embodiment of the present invention;
FIG. 6 is a front section of the cap;
FIG. 7 is a side section of the cap;
FIGS. 8 and 9 are front section and side section showing a cap in accordance with a modification of the first embodiment;
FIG. 10 is a front section showing a cap in accordance with another modification of the first embodiment;
FIG. 11 is a perspective view of a cap in accordance with a second embodiment of the present invention;
FIGS. 12 and 13 are horizontal section and front section showing the cap;
FIG. 14 is a cross section taken along line X-X of FIG. 13;
FIGS. 15 and 16 are horizontal section and front section showing a cap in accordance with a third embodiment of the present invention;
FIG. 17 is a front section of a cap in accordance with a fourth embodiment of the present invention;
FIG. 18 is a front section of a cap in accordance with a modification of the fourth embodiment;
FIG. 19 is a front section of a cap in accordance with a fifth embodiment of the present invention;
FIGS. 20 to 22 are views explaining how to apply the mascara composition while curing the eyelashes with the use of the device; and
FIGS. 23 and 24 are exploded perspective views of another eyelash treatment device which can be equally utilized in the present invention;
FIG. 25 is a partial top view of the applicator of the above device; and
FIG. 26 is a cross section of the applicator.

### DETAILED DESCRIPTION OF THE PREFERRED EMBODIMENT

While the description concludes with claims particularly pointing out and distinctly claiming the invention, it is believed that the present invention will be better understood from the following description.

A product in accordance with the present invention is a combination of a mascara composition and an eyelash treatment device.

### Mascara Treatment device

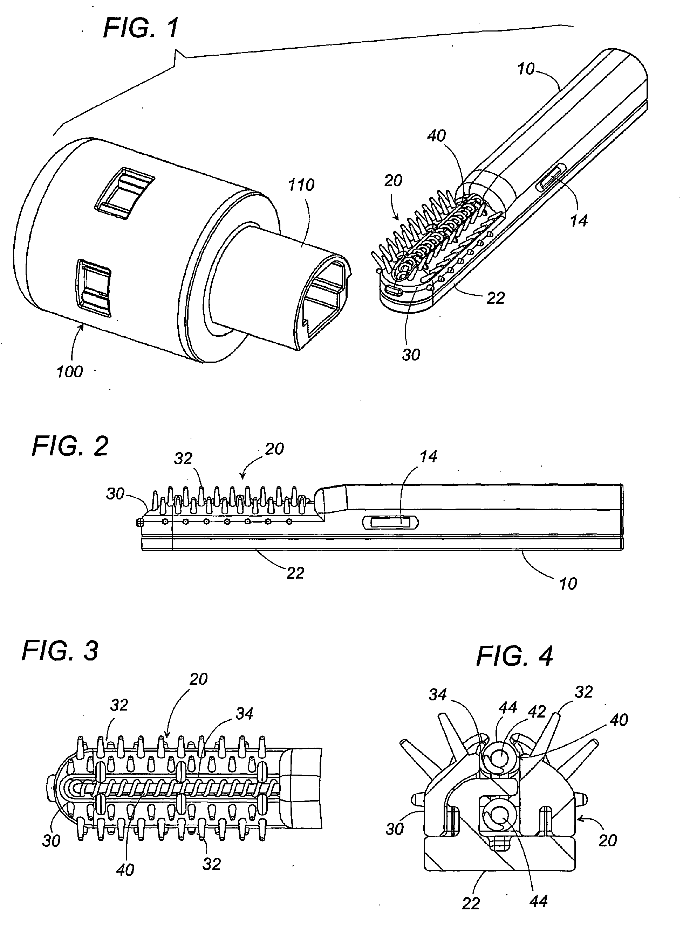

Now referring to FIGS. 1 to 4, there is shown an eyelash treatment device in accordance with a preferred embodiment of the present invention. The device uses a solid mascara composition which is solid at room temperature, namely 25 ºC, and is softened with elevated temperature such that it can be applied to the eyelashes. By "solid" herein for describing the mascara composition, what is meant is that the composition has a certain hardness to retain its structure, and also that the composition is stable against stress or shear. The mascara composition goes through a transition change in terms of rheology between 25 ºC and 100 ºC, such that during these temperatures, there is a range of temperature and rheology in which the composition is suitably softened for application to the eyelashes. The softened state of the mascara composition is fluid enough to be applied to the eyelashes, however, is viscous enough to stay on the applicator upon application, and on the eyelashes after application.

The device includes an elongated hand grip **10** carrying at its one longitudinal end an applicator **20** for applying the softened mascara composition to user's eyelashes. The applicator **20** is elongated to have a length generally aligned with a length of the grip **10,** and includes a heater **40** which is configured to heat the mascara composition for softening the composition after it is loaded to the applicator **20** on one hand, and to heat the eyelashes for curling on the other hand. The mascara composition is provided in the form of a solid piece and is held in a cap **100** detachable to the applicator. The cap **100** is configured to include a loading mechanism for loading or delivering the mascara piece one by one onto the applicator, details of which will be discussed later.

The applicator **20** is composed of a head **22** extending integrally from the grip **10,** and a comb attachment **30** detachably fitted on the head **22.** The head **22** is made of a dielectric plastic material and carries the heater **40** composed of a resistor coil **42** wound around a U-shaped core **44** of dielectric material to give two parallel vertical rows running in the length of the applicator **20.** The coil **42** is electrically connected to a voltage source, i.e., a battery within the grip **10** and is energized by manipulating a switch handle **14** on the side of the grip **10.** The comb attachment **30** is made of a dielectric plastic material and is fitted on the head **22** in thermal transfer relation with the heater **40** so as to be heated to an elevated temperature for softening the mascara composition. The comb attachment **30** is shaped to have a rounded top face provided with a comb having a plurality of comb teeth **32** which are arranged along the length and width of the applicator for applying the softened mascara composition to the eyelashes while smoothening the eyelashes. A ditch **34** is formed in the width center of the top face to extend the length of the applicator **20** for receiving an upper part of the heater **40** and also for providing a space within which the softened mascara composition can be retained. The softened mascara composition is thereafter allowed to flow over the comb teeth, climbing-up to the comb teeth **32** by the action of a surface tension to be ready for being delivered to the eyelashes as the comb teeth **32** smoothen the eyelashes.

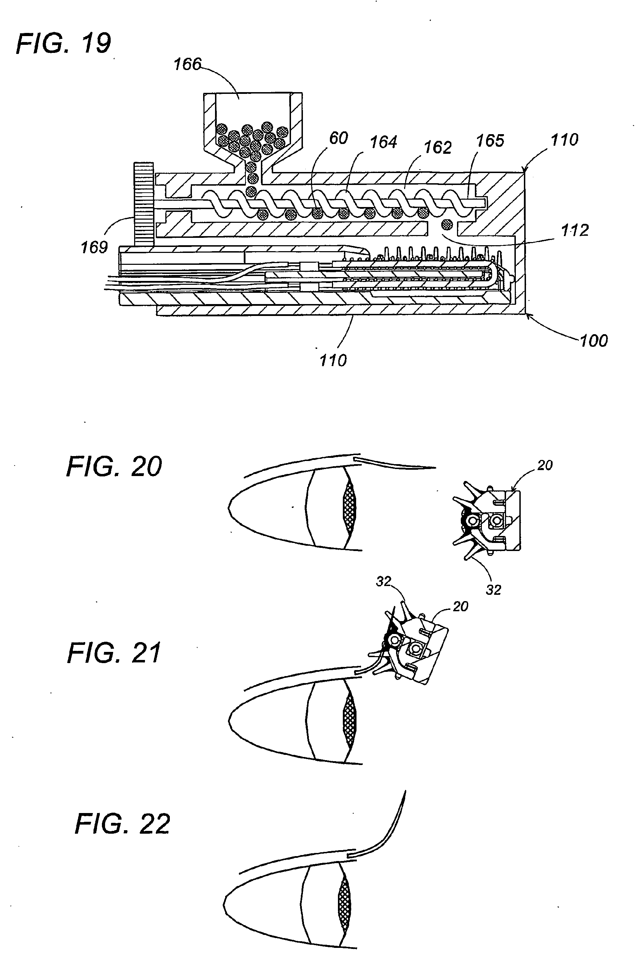

In operation, the applicator **20** is firstly placed in a position with the comb attachment **30** just below the eyelashes, as shown in FIG. 20. Then, the applicator **20** is raised and twisted to some extent for smoothing the eyelashes with the comb teeth **32,** as shown in FIG. 21, thereby applying the softened mascara composition to the eyelashes, while lifting the same. In this condition, the top face of the comb attachment **30** comes into contact with the eyelashes for heating and curling the eyelashes. As soon as the applicator **20** is moved away from the eyelashes, as shown in FIG. 22, the softened mascara composition is cooled quickly to give a firm film of the solidified mascara composition on the eyelashes. Thus, the above single operation can give the effect of forming the mascara film as well as curling the eyelashes.

The heater **40** is controlled to heat the comb attachment **30** to a temperature of about 50 °C to 100 °C for softening the mascara composition. At the elevated temperature, the softened mascara composition exhibits a viscosity of 1 mPas to 10,000,000 mPas, sufficient for coating the eyelashes, but being kept from flowing out of the applicator **20** for assuring a safe application of the mascara composition.

### Mascara Composition

All percentages, parts and ratios are based upon the total weight of the compositions of the present invention, unless otherwise specified. All such weights as they pertain to listed ingredients are based on the active level and, therefore do not include carriers or by-products that may be included in commercially available materials.

All ingredients such as actives and other ingredients useful herein may be categorized or described by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

The mascara composition of the present invention is solid at room temperature, namely 25ºC, and is softened with elevated temperature such that it can be applied to the eyelashes. By "solid" herein for describing the mascara composition, what is meant is that the composition has a certain hardness to retain its structure, and also that the composition is stable against stress or shear. The present composition has a needle penetration, as measured according to the American Standard prescribed by the American Society for Testing and Materials (ASTM) Test Method D5, of from about 1 to about 40, and a yield stress of no less than about 1500 Pa, both measurements at 25 ºC, preferably at 35ºC.

The principle of the measurement of the needle penetration according to the ASTM D5 consists of measuring the depth, expressed in tenths of a millimeter, to which a standard needle (weighing 2.5 g and placed in a needle holder weighing 47.5 g, i.e., a total of 50 g) penetrates when placed on the composition for 5 seconds. The principle of the measurement of yield stress consists of measuring oscillation stress sweep for understanding flow behavior and viscoelastic character for fluids and semi-solids as a function of stress, shear rate, or temperature. In the present invention, the yield stress is measured using a TA Instrument Rheometer AR-500 using a 40 mm Al Parallel Plate (Gap: 600 mm) at 1Hz. At 25 ºC, the present composition preferably has no measurable yield point under stress or shear.

The mascara composition changes rheology as it is heated, and finally reaches a point where it is liquid by 100 ºC, preferably by 90 ºC. By "liquid" herein for describing the mascara composition, what is meant is that the composition has a viscosity of between about 1 mPas and about 10,000,000 mPas. The present mascara composition goes through a transition change in terms of rheology between 25 ºC and 100 ºC, such that during these temperatures, there is a range of temperature and rheology in which the composition is suitably softened for application to the eyelashes. The softened state of the mascara composition is fluid enough to be applied to the eyelashes with an average number of strokes of an applicator holding the composition, however, is viscous enough to stay on the applicator upon application, and on the eyelashes after application. When the composition is too thin or watery, the composition is difficult to hold on the applicator, and amount of composition applied to the eyelashes decreases.

The mascara composition is designed to have a rheology profile which gives suitable rheology during the temperature band to which the eyelash treatment device is heated. The temperature band may be selected to provide suitable balance of applicability of the mascara composition and eyelash curling/lifting benefit. Typically, the temperature band is between about 50ºC and about 100ºC.

The components for the composition are selected in order to provide the desired rheology profile. The composition comprises at least a solid hydrophobic component for providing the essential physical characteristics of the present invention. The composition may be made solely by the solid hydrophobic component.

The composition may take the phase form of an oil mixture, the oil being mainly made by the wax, or a water-in-oil emulsion. Water-in-oil emulsion forms are suitable for encompassing water-soluble or water-dispersible components.

Alternatively, the mascara composition may comprise an outer shell comprising at least a solid hydrophobic component having the unique rheological profiles described above, and an inner core which has a certain viscosity. The mascara composition in room temperature is in solid form encasing a fluid inner core. After heating, such embodiment composition is believed to provide an adequate crystallization time before solidifying, thereby allowing the user to have adequate time to apply and fix the composition on the eyelashes. Such longer "play time" allows the user to achieve the desired appearance of eyelashes.

The inner core may be an aqueous continuous composition containing 15% to 95% water by weight of the inner core. Such aqueous continuous phase inner core is easy to remove with soap and water. The inner core may also be an oil continuous composition containing 30% to 50% volatile liquid oil by weight of the inner core. Such oil continuous phase inner core provides waterproof benefit.

### Solid Hydrophobic Component

The present composition comprises a solid hydrophobic component for providing the solid characteristic of the mascara composition. Solid hydrophobic components are typically used at levels from about 25% to about 100% in oil mixture forms, and from about 25% to about 95% in water-in-oil emulsion forms. Suitable solid hydrophobic components include waxes and fats.

Waxes are defined as lower-melting organic mixtures or compounds of high molecular weight, solid at room temperature and generally similar in composition to fats and oils except that they contain no glycerides. Some are hydrocarbons, others are esters of fatty acids and alcohols. Waxes useful in the present invention are selected from the group consisting of animal waxes, vegetable waxes, mineral waxes, synthetic waxes petroleum waxes, ethylenic polymers, hydrocarbon types such as Fischer-Tropsch waxes, silicone waxes, and mixtures thereof wherein the waxes have a melting point between 25°C and 100°C.

The specific waxes useful in the present invention are selected from the group consisting of beeswax, lanolin wax, shellac wax (animal waxes); carnauba, candelilla, bayberry (vegetable waxes); ozokerite, ceresin, (mineral waxes); paraffin, microcrystalline waxes (petroleum waxes); polyethylene, (ethylenic polymers); polyethylene homopolymers (Fischer-Tropsch waxes); C24-45 alkyl methicones (silicone waxes); and mixtures thereof.

Highly preferable commercially available waxes herein include stearyl palmitate by the tradename PURESTER 34, available from Strahl & Pitsch, ceresin by the tradename CERESIN 252 available from Strahl & Pitsch, and paraffin wax by the tradenames PARAFFIN SP-673P, PARAFFIN 206, and PARAFFIN 192 available from Strahl & Pitsch.

Useful herein are fats, namely glyceryl esters of higher fatty acids such as stearic and palmitic. Such esters and their mixtures are solid at room temperature and exhibit crystalline structure. The fats employed according to the invention are selected from the group consisting of fats derived from animals, vegetables, synthetically derived fats, and mixtures thereof wherein said fats have a melting point from about 55°C to about 100°C. Preferably, the fats are selected from the group consisting of glyceryl monostearate, glyceryl distearate, glyceryl tristearate, palmitate esters of glycerol, C18-36 triglycerides, glyceryl tribehenate, C18-36 acid triglycerides and mixtures thereof.

Highly preferable commercially available fats herein include glyceryl monostearate by the tradename CUTINA GMS-V available from Cognis Cutina.

### Pigments

The compositions of the preset invention may comprise pigments selected from the group consisting of inorganic pigments, organic pigments, and organic lake pigments, pearlescent pigments, and mixtures thereof. The present invention comprises mascara compositions devoid of pigments however, as such compositions may also provide the benefits of the present invention.

When employed, the pigments are present in proportions depending on the color and the intensity of the color that it is intended to produce. When employed, the level of pigments in the composition is from about 3 % to about 25 %, preferably from about 5 % to about 15 %. The pigments may optionally be surface-treated with treatments that include, but are not limited to, silicones, perfluorinated compounds, lecithin, and amino acids.

Inorganic pigments useful in the present invention include those selected from the group consisting of rutile titanium dioxide, anatase titanium dioxide (both coded in the Color Index under the reference CI 77891); black, yellow and red iron oxides (CI 77499, 77492 and 77491); bismuth oxychloride (CI 77163); manganese violet (CI 77742); ultramarines (CI 77007); chromium oxide (CI 77288); chromium hydroxide (CI 77289); ferric ferrocyanide (CI 77510); zinc oxide (CI 77947); and mixtures thereof.

The organic pigments useful in the present invention include carbons black, and the dyes and the analogous lakes selected from the group consisting of D&C Red 6 (CI 15850); D&C Red 7 (CI 15850:1); D&C Red 21 (CI 45380:2); D&C Red 22 (CI 45380); D&C Red 27 (CI 45410:1); D&C Red 28 (CI 45410); D&C Red 30 (CI 73360); D&C Red 33 (CI 17200); D&C Red 34 (CI 15880:1); D&C Red 36 (CI 12085); D&C Orange 4 (CI 15510); D&C Orange 5 (CI 45370:1); D&C Orange 11 (CI 45425); FD&C Yellow 5 (CI 19140), FD&C Yellow 6 (CI 15985); D&C Yellow 10 (CI 47005); FD&C Green 3 (CI 42053); D&C Green 5 (CI 61570); FD&C Blue 1 (CI 42090); Cochineal Carmine (CI 75470); Guanine (CI 75170) and mixtures thereof.

The pearlescent pigments useful in the present invention include those selected from the group consisting of mica (or a similar plate-like substrate) coated with any of the following materials alone or in combination: titanium dioxide, bismuth oxychloride, iron oxides, ferric ferrocyanide, chromium oxide, chromium hydroxide, and any organic pigment of the above-mentioned type and mixtures thereof.

### Film Forming Polymer

The compositions of the present invention may comprise a film forming polymer, for imparting wear and/or transfer resistant properties. When included, such materials are typically used in an amount of from about 0.5 % to about 20 % preferably from about 0.5% to about 10% by weight of the composition. Preferred polymers form a non-tacky film which is removable with water used with cleansers such as soap. The film forming polymers herein can be hydrophobic or hydrophilic, and can be provided in a lipophilic or aqueous carrier. When polymers provided in aqueous carriers are employed in the composition, a water-in-oil form is selected. Polymers of hydrophilic nature are also compatible with a water-in-oil form composition.

Examples of suitable film forming polymeric materials include:
a) sulfopolyester resins, such as those with tradename AQ sulfopolyester resins, such as AQ29D, AQ35S, AQ38D, AQ38S, AQ48S, and AQ55S available from Eastman Chemicals;
b) polyvinylacetate/polyvinyl alcohol polymers, such as tradename Vinex resins available from Air Products, including Vinex 2034, Vinex 2144, and Vinex 2019;
c) acrylic resins, including water dispersible acrylic resins available from National Starch under the trade name "Dermacryl", including Dermacryl LT;
d) acrylates and their derivative polymers, including acrylates copolymer with tradename Luvimer available from BASF, Avalure series available from Noveon, Daitosol 5000AD available from Daito Kasei Kogyo, ethyleneatyrene/acrylates copolymer such as Syntran series available from Interpolymer, acrylates/ammonium methacrylate copolymer with tradename Ultrasol 2075C available from Presperse, octyl acrylates copolymer with tradename Daitotol SJ available from Kobo, acrylates silicone copolymer with tradename Daitotol ASC available from Kobo, AMP-acrylates/allyl methacrylate copolymer with tradename Fixate G100 Polymer available from Noveon, acrylate/dimethicone copolymer with tradename KP545 available from ShinEtsu;
e) styrene, such as sodium polystyrene sulfonate with tradename Flexan available from National Starch;
f) urethanes, such as polyurethane-1 polymer with tradename Luviset PUR available from BASF;
g) polyvinylpyrrolidones (PVP), including tradenames Luviskol K17, K30 and K90 available from BASF PVP K-30, PVP K-120 available from ISP, tricontanyl PVP with tradename Ganex WP 660 Resin available from ISP, water soluble copolymers of PVP, including PVP/VA S-630 and W-735 and PVP/dimethylaminoethylmethacrylate Copolymers such as Copolymer 845, Copolymer 937, and Styleze CC-10 available from ISP, VP/DAM available from Daiichi Kogyo Seiyaku, PVP/acrylates/lauryl methacrylate copolymer with tradename Styleze 2000 available from ISP;
h) high molecular weight silicones such as dimethicone and organic-substituted dimethicones, especially those with viscosities of greater than about 50,000 mPas;
i) high molecular weight hydrocarbon polymers with viscosities of greater than about 50,000 mPas such as polybutene, polybutene terephthalate, polydecene, polycyclopentadiene, and similar linear and branched high molecular weight hydrocarbons, including isooctane with tradename Permethyl 97A available from Presperse;
j) organosiloxanes, including organosiloxane resins, fluid diorganopolysiloxane polymers and silicone ester waxes.

Also useful herein are latex polymers including copolymer PVP/hexadecane or the copolymer PVP/eicosene marketed by ISP under the tradenames Ganex V-216® and Ganex V-220®, respectively. Ganex V-216® is a PVP/hexadecane copolymer comprising approximately 15-23% of pyrrolidone units with a weight average molecular weight of 7300. Ganex V-220® is a copolymer PVP/eicosene which comprises approximately 20-28% of pyrrolidone units and a weight average molecular weight of 8600.

### Emulsifiers

The compositions of the present invention in emulsion form comprises an emulsifier, which is typically a lipophilic surfactant, preferably by weight of the entire composition at from about 1 % to about 15 %. The lipophilic surfactant herein has an HLB value of less than about 8.

The HLB value is a theoretical index value which describes the hydrophilicity-hydrophobicity balance of a specific compound. Generally, it is recognized that the HLB index ranges from 0 (very hydrophobic) to 40 (very hydrophilic). The HLB value of the lipophilic surfactants may be found in tables and charts known in the art, or may be calculated with the following general equation: HLB = 7 + (hydrophobic group values) + (hydrophilic group values). The HLB and methods for calculating the HLB of a compound are explained in detail in Surfactant Science Series, Vol. 1: Nonionic Surfactants", pp 606-13, M. J. Schick (Marcel Dekker Inc., New York, 1966).

Without being bound by theory, the species and levels of the lipophilic surfactant herein are believed to provide a stable water-in-oil emulsion in view of the other components of the present invention.

The lipophilic surfactant can be an ester-type surfactant. Ester-type surfactants useful herein include: sorbitan monoisostearate, sorbitan diisostearate, sorbitan sesquiisostearate, sorbitan monooleate, sorbitan dioleate, sorbitan sesquioleate, glyceryl monoisostearate, glyceryl diiostearate, glyceryl sesquiisostearate, glyceryl monooleate, glyceryl dioleate, glyceryl sesquioleate, diglyceryl diisostearate, diglyceryl dioleate, diglycerin monoisostearyl ether, diglycerin diisostearyl ether, and mixtures thereof.

Commercially available ester-type surfactants are, for example, sorbitan isostearate having a tradename Crill 6 available from Croda, and sorbitan sesquioleate with tradename Arlacel 83 available from Kao Atras.

The lipophilic surfactant can be a silicone-type surfactant. Silicone-type surfactants useful herein are (i), (ii), and (iii) as shown below, and mixtures thereof.
(i) dimethicone copolyols having the formulation: 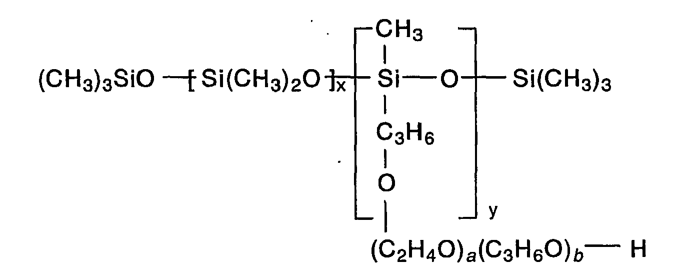 wherein x is an integer from 5 to 100, y is an integer from 1 to 50, a is zero or greater, b is zero or greater, the average sum of a+b being 1-100.
(ii) dimethicone copolyols having the formulation: 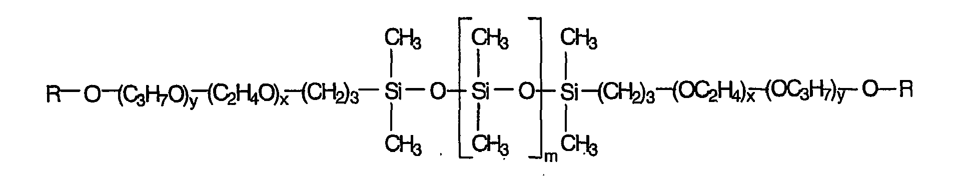 wherein R is selected from the group consisting of hydrogen, methyl, and combinations thereof, m is an integer from 5 to 100, x is independently zero or greater, y is independently zero or greater, the sum of x+y being 1-100.
(iii) branched polyether-polydiorganosiloxane emulsifiers herein having the formulation: 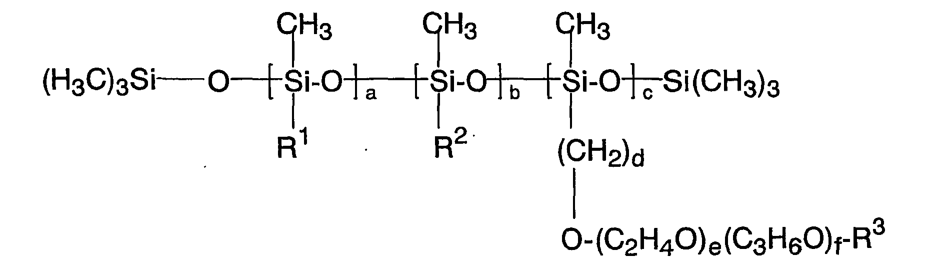 wherein R¹ is an alkyl group having from about 1 to about 20 carbons; R² is 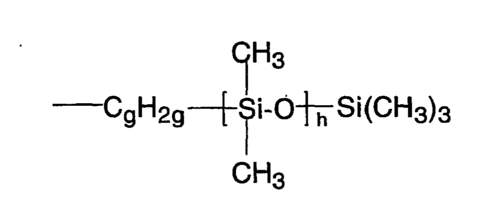
wherein g is from about 1 to about 5, and h is from about 5 to about 20; R³ is H or an alkyl group having from about 1 to about 5 carbons; e is from about 5 to about 20; f is from about 0 to about 10; a is from about 20 to about 100; b is from about 1 to about 15; c is from about 1 to about 15; and d is from about 1 to about 5.

Commercially available silicone-type surfactants are, for example, dimethicone copolyols DC5225C, BY22-012, BY22-008, SH3746M, SH3771 M, SH3772M, SH3773M, SH3775M, SH3748, SH3749, and DC5200, all available from Dow Corning, and branched polyether-polydiorganosiloxane emulsifiers such as PEG-9 polydimethylsiloxyethyl Dimethicone, having an HLB of about 4 and a molecular weight of about 6,000 having a tradename KF 6028 available from ShinEtsu Chemical.

### Water

The composition of the present invention in water-in-oil form comprises water in an amount sufficient to provide a discontinuous aqueous phase, preferably an amount such that water is no more than about 50 %, more preferably from about 10 % to about 40 % of the entire composition. Use of water allows the inclusion of useful components such as film forming polymers which are hydrophilic and/or aqueous carrier-based, hydrophilic conditioning agents, and other water soluble or water dispersible components described below.

In the present invention, deionized water is typically used. Water from natural sources including mineral cations can also be used, depending on the desired characteristic of the product.

### Hydrophobic Conditioning Agents

The compositions of the present invention may further comprise a hydrophobic conditioning agent. Nonlimiting examples of hydrophobic conditioning agents include those selected from the group consisting of mineral oil, petrolatum, lecithin, hydrogenated lecithin, lanolin, lanolin derivatives, C7-C40 branched chain hydrocarbons, C1-C30 alcohol esters of C1-C30 carboxylic acids, C1-C30 alcohol esters of C2-C30 dicarboxylic acids, monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, propylene glycol diesters of C1-C30 carboxylic acids, C1-C30 carboxylic acid monoesters and polyesters of sugars, polydialkylsiloxanes, polydiarylsiloxanes, polyalkarylsiloxanes, cyclomethicones having 3 to 9 silicon atoms, polysiloxane crosspolymers such as vinyl dimethicone crosspolymer available as a dimethicone mixture fluid with tradename KSG series available from ShinEtsu, vegetable oils, hydrogenated vegetable oils, polypropylene glycol C4-C20 alkyl ethers, di C8-C30 alkyl ethers, and combinations thereof.

### Hydrophilic Conditioning Agents

The compositions of the present invention may further comprise a hydrophilic conditioning agent. Nonlimiting examples of hydrophilic conditioning agents include those selected from the group consisting of polyhydric alcohols, polypropylene glycols, polyethylene glycols, ureas, pyrolidone carboxylic acids, ethoxylated and/or propoxylated C3-C6 diols and triols, alpha-hydroxy C2-C6 carboxylic acids, ethoxylated and/or propoxylated sugars, polyacrylic acid copolymers, sugars having up to about 12 carbons atoms, sugar alcohols having up to about 12 carbon atoms, and mixtures thereof.

### Solvents

The compositions of the present invention may contain a volatile or non-volatile solvent that dissolves or uniformly disperses certain components of the present invention. They include, but are not limited to, lower alcohols (such as ethanol, isopropanol), dihydric alcohols such as propylene and butylene glycol, polyols such as glycerin, hydroalcoholic mixtures, hydrocarbons (such as isobutane, hexane, decene, acetone), halogenated hydrocarbons (like Freon), linalool, hydrocarbon esters (such as ethyl acetate, dibutyl phthalate), volatile silicon derivatives, especially siloxanes (such as phenyl pentamethyl disiloxane, phenethyl pentamethyl disiloxane, methoxypropyl heptamethyl cyclotetrasiloxane, chloropropyl pentamethyl disiloxane, hydroxypropyl pentamethyl disiloxane, octamethyl cyclotetrasiloxane, decamethyl cyclopentasiloxane), and mixtures thereof.

### Additional components

The compositions hereof may further contain additional components such as are conventionally used in topical products, e.g., for providing aesthetic or functional benefit to the composition or skin, such as sensory benefits relating to appearance, smell, or feel, therapeutic benefits, or prophylactic benefits (it is to be understood that the above-described required materials may themselves provide such benefits).

The CTFA Cosmetic Ingredient Handbook, Second Edition (1992) describes a wide variety of nonlimiting cosmetic and pharmaceutical ingredients commonly used in the industry, which are suitable for use in the topical compositions of the present invention. Such other materials may be dissolved or dispersed in the composition, depending on the relative solubilities of the components of the composition.

Examples of suitable topical ingredient classes include: sunscreen actives, anti-cellulite agents, antioxidants, radical scavengers, chelating agents, vitamins and derivatives thereof, abrasives, other oil absorbents, astringents, dyes, essential oils, fragrance, structuring agents, emulsifiers, solubilizing agents, anti-caking agents, antifoaming agents, binders, buffering agents, bulking agents, denaturants, pH adjusters, propellants, reducing agents, sequestrants, cosmetic biocides, and preservatives, such as propylparaben, methyl paraben, phenoxyethanol, benzyl alcohol, and EDTA and its salts.

### Additional Usages

The cosmetic products herein may also be used for other usages in the personal care field, with necessary modifications to the composition and/or device suitable for the usage. Unlimited examples of such usages include coloring and treatment of eyebrows; treatment, styling, removing, and coloring of hair; treatment and tattooing of skin, and nail coloring.

### EXAMPLES

The following examples further describe and demonstrate the preferred embodiments within the scope of the present invention. The examples are given solely for the purpose of illustration, and are not to be construed as limitations of the present invention since many variations thereof are possible without departing from its spirit and scope.

### Examples C1-C3

Examples C1 and C2 are mascara compositions in the form of oil mixture, and C3 is a mascara composition in the form of a water-in-oil emulsion. Each composition has a needle penetration of between 1-25 at 35ºC, a yield stress of over 1500Pa at 35ºC, and has a viscosity of between 1 mPas and 10,000,000 mPas at 90ºC. The mascara compositions can be suitably applied to the eyelashes when heated to between 50-80ºC via use of the device described hereinabove.

### Composition

The following components are used at the respective w/w %.

| No | Component | C1 | C2 | C3 |
|---|---|---|---|---|
| 1 | Glyceryl Monostearate-Vegetable Derived *1 | 8.00 | 10.00 | 10.00 |
| 2 | Stearyl Palmitate *2 | 26.00 | 18.00 | 17.00 |
| 3 | Ceresine Wax *3 | 26.00 | 18.00 | 17.00 |
| 4 | Paraffin Wax *4 | 15.50 | 9.70 | 7.87 |
| 5 | Dimethicone/Vinyl Dimethicone Crosspolymer *5 | | 25.00 | |
| 6 | Tricontanyl PVP *6 | 2.00 | 2.00 | 2.00 |
| 7 | Sorbitan Sesquioleate *7 | | | 5.00 |
| 8 | Propylparaben | 0.30 | 0.30 | 0.10 |
| 9 | CI 77499 (iron oxide) and Methicone *8 | 22.20 | 17.00 | 15.00 |
| 10 | Deionized Water | | | 20.00 |
| 11 | Sodium Polystyrene Sulfonate *9 | | | 5.00 |
| 12 | Phenoxyethanol | | | 0.30 |
| 13 | Methylparaben | | | 0.20 |
| 14 | Benzyl alcohol | | | 0.40 |
| 15 | Trisodium EDTA | | | 0.13 |

### Definition of components:

*1 Glyceryl monostearate-vegetable derived: GMS-V available from Cognis
*2 Stearyl Palmitate: Purester 34 available from Strahl & Pitsch
*3 Ceresine wax: Ceresine Wax SP-252 available from Strahl & Pitsch
*4 Paraffin wax: Paraffin Wax SP-673P available from Strahl & Pitsch
*5 Dimethicone/Vinyl Dimethicone Crosspolymer: KSG 16 available from ShinEtsu
*6 Tricontanyl PVP: Ganex WP-660 available from ISP
*7 Sorbitan Sesquioleate: Crill 6 available from Croda
*8 CI 77499 (iron oxide) and Methicone: Si Black Iron Oxide available from Daito Kasei
*9 Sodium polystyrene sulfonate: Flexan II available from National Starch & Chemical

### Method of preparation

Examples C1-C3 may be made in any suitable method known to one skilled in the art. Preferably, the examples are made by the following methods.

### Example C1

1) Heat components 1-4 to 85-90 °C. Being low shear mixing when enough wax has melted.
2) Once components 1-4 have completely melted, add components 6 and 8. Continue mixing for about 10 min.
3) Add component 9 to product of Step 2, and disperse for 30 min - 1 hr with a dispersator.
4) Pour the product of Step 3 into molds and allow product to cool and solidify.

### Example C2

1) Heat components 1-4 to 85-90°C. Being low shear mixing when enough wax has melted.
2) Once components 1-4 has completely melted, add component 5. Continue mixing for about 10 min.
3) Add components 6 and 8 to product of Step 2. Continue mixing for about 10 min.
4) Add component 9 to product of Step 3, and disperse for 30 min - 1 hr with a dispersator.
5) Pour the product of Step 4 into molds and allow product to cool and solidify.

### Example C3

1) Heat components 1-4 to 85-90°C. Being low shear mixing when enough wax has melted.
2) Separately heat components 10-15 to 85-90°C and mix with low shear mixing.
3) Once components 1-4 has completely melted, add components 6-8. Continue mixing for about 10 min.
4) Add component 9 to product of Step 3, and disperse for 30 main - 1 hr with a dispersator 2. Homogenize for 30 min -1 hr by mixing.
5) Add the product of Step 2 to product of Step 4 while mixing with low shear mixing.
6) Further mix product of Step 5 with moderate shear mixing for 15 - 30 min. to effect emulsification.
7) Pour the product of Step 6 into molds and allow product to cool and solidify.

### Examples C4-C5

Examples C4 and C5 are mascara compositions in the form of a hard core shell made by the composition of C1 described above, and inner core compositions as shown below. C4 is an aqueous continuous phase composition, and C5 is an oil continuous phase composition.

| Component | Inner core for C4 | Inner core for C5 |
|---|---|---|
| Paraffin wax | 2 | 10 |
| Bees wax | 2 | 5 |
| Carnauba wax | 4 | 5 |
| Synthetic wax | 6 | |
| Polyethylene wax | | 2 |
| Ceresin | | 5 |
| Trihydroxystearin | | 2 |
| Quaternium-18 hectorite | 5 | 8 |
| Propylene carbonate | 1 | 3 |
| Glyceryl monostearate | 7.5 | |
| Stearic acid | 1.7 | |
| Triethanolamine | 1.5 | |
| Polyvinyl alcohol | 1.5 | |
| Iron oxide | 10 | 12 |
| Propylparaben | 0.2 | 0.2 |
| Methylparaben | 0.2 | |
| Ethyl alcohol | 10 | |
| Water | 35.3 | |
| Acrylate Copolymer emulsion | 20 | |
| Volatile isoparaffin | | 37.8 |
| High molecular weight Dimethicone | | 10 |

### Loading Mechanism of Mascara Treatment device

Now, the details of the loading mechanism utilized in the present invention will be discussed with reference to several embodiments and their modifications.

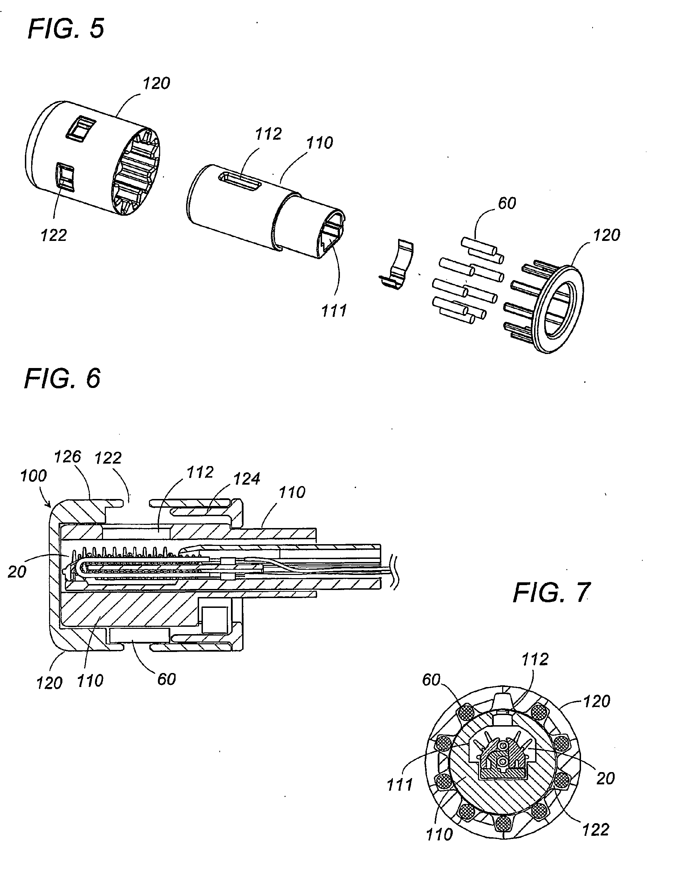

### First Embodiment <FIGS. 5 to 7>

Referring to FIGS. 5 to 7, there is shown a first embodiment of the loading mechanism provided on the side of the cap **100.** The cap **100** includes a sheath **110** surrounding the applicator **20,** and a carrier **120.** The sheath **110** is formed with an opening **112** which comes into an open communication with the width center of the applicator **20** when the cap **100** is fitted over the applicator **60.** The sheath **110** is shaped to have a non-circular bore **111** within which the applicator **20** is slidable but is rotatively fixed so that the applicator **20** has its widthwise center held into constant confrontation with the opening **112.** The carrier **120** is in the form of a sleeve which is coaxial with the sheath **110** and is rotatable relative to the sheath **110.** The carrier **120** is formed with a plurality of circumferentially spaced compartments **122** each storing the mascara piece **60.** The carrier **120** is rotatable relative to the sheath to make one of the compartments **122** selectively in registration with the opening for allowing the mascara piece **60** to drop onto the applicator **20** through the opening **112,** thereby loading the mascara piece one by one to the applicator **20.** The carrier **120** is composed of a rotating shell **124** rotatably coupled to the sheath **110,** and a jacket **126** forming outer bottoms of the compartments **122.** The compartments **122** are defined between the shell **124** and the jacket **126.** The jacket **126** may be configured to be detachable to the shell **124,** enabling to refill the mascara pieces by removal of the jacket **126.**

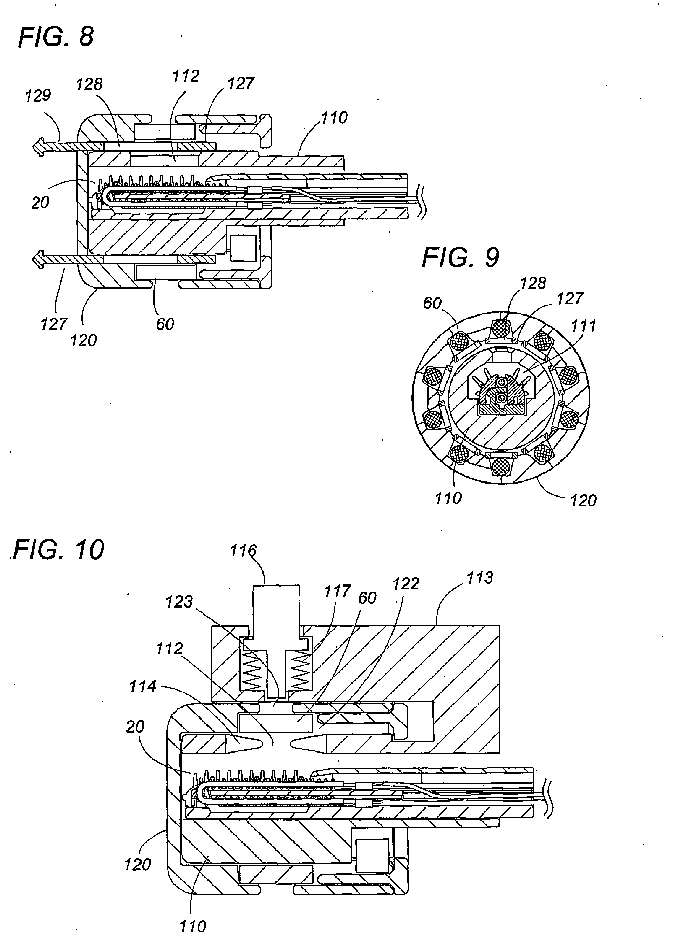

FIGS. 8 and 9 show a modification of the above loading mechanism which is identical to the above embodiment except for the provision of a gate **127** to each of the compartments **122.** Each gate **127** is held slidable in the axial direction of the carrier **120,** and has its one end projected beyond the end of the carrier **120** to define thereat an operator knob **129.** The gate **127** has a through-hole **128** and is normally held in a closing position of closing the associated compartment **122** for prohibiting the mascara piece **60** from dropping through the opening to the applicator **20.** When the gate **127** is pushed axially inward, the compartment **122** is opened to allow the mascara piece **60** to drop by way of the through-hole **128** and the opening **112** to the applicator **20.** Alternatively, it may be arranged that a single gate is provided on the side of the carrier **110** rather than the carrier **120.**

FIG. 10 shows another modification of the above loading mechanism which is identical to the above embodiment except for a provision of a plunger **116** which is configured to force the mascara piece **60** out of the compartment **122.** The opening **112** is equipped with a normally-closed flap valve **114** for retaining the mascara piece **60** within the compartment **122** even when any one of the compartment comes into registration with the opening **112.** The plunger **116** is supported to an extension **113** of the sheath **112** so as to be radially movable relative to the sheath **110** for advancing its inner radial end for pushing contact with the mascara piece **60** through an aperture **123** in the exterior wall of the carrier **120.** When the plunger **116** is pushed against a bias of spring **117,** it forces the mascara piece **60** radially inward, thereby temporarily opening the flap valve **114** and accordingly loading the mascara piece **60** to the applicator **20.** Upon releasing the plunger **116** after loading the mascara piece **60,** the flap valve **114** returns by its resiliency to the closed position.

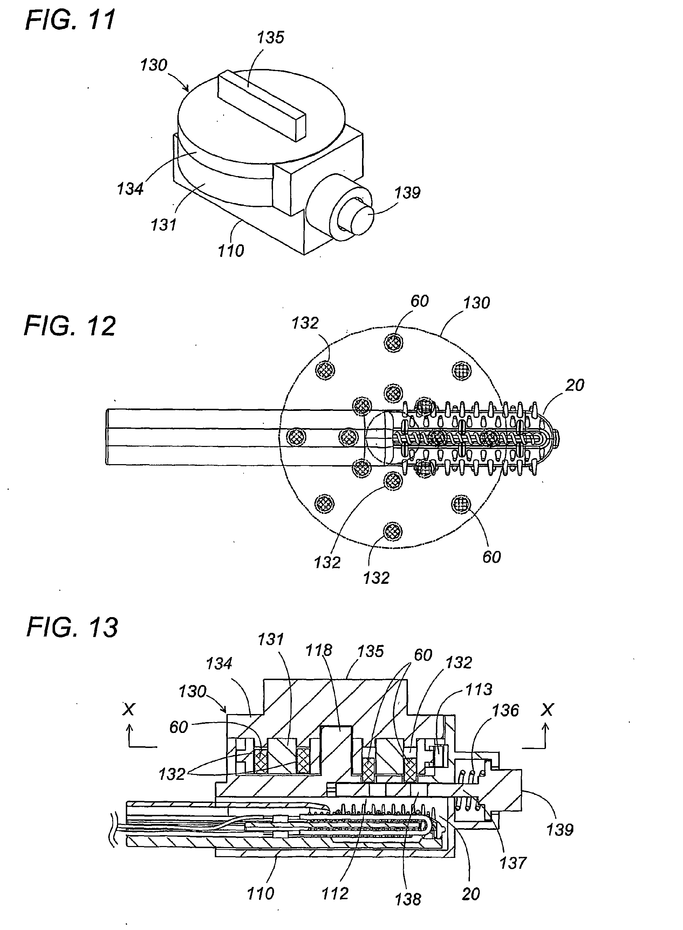

### Second Embodiment <FIGS. 11 to 14>

FIGS. 11 to 14 illustrate a second embodiment of the loading mechanism which is similar to the above embodiment except for the provision of the carrier in the form of a disc **130** which is supported to the sheath **110** to be rotatable relative thereto about an axis perpendicular to the lengthwise axis of the sheath **110.** The disc **130** is composed of a base **131** and a jacket **134** which are secured together to define therebetween a plurality of circumferentially spaced compartments **132** for holding the mascara pieces **60,** respectively. The disc **130** is mounted around a spindle **118** integrally projecting on top of the sheath **110** to be rotatable about the axis of the stud. The compartments **132** are arranged into two circumferential arrays such that each compartment in one of the arrays is radially aligned with each corresponding one in the other array in order to deliver the two mascara pieces **60** simultaneously to the two longitudinally spaced spots on the applicator **20** through the opening **112** elongated axially along the axis of the sheath **110.** The compartments **132** are open in the lower surface of the disc **130** to be in communication with the opening **112** in the sheath **110.** The sheath **110** is additionally formed with a gate **137** slidable along the axis of the sheath **110** so as to normally close the bottoms of the compartments **132** coming into registration with the opening **112,** retaining the mascara pieces within the disc **130.** The gate **137** is formed at its lengthwise end with an operation knob **139** to be accessible by a user's finger. When the knob **139** is pressed against a bias of a spring **136** to move the gate axially inwardly, through-holes **138** in the gate come into open communication with the compartments **132,** thereby permitting the mascara pieces **60** to drop onto the applicator **20** by way of the through-holes **138** and the opening **112.**

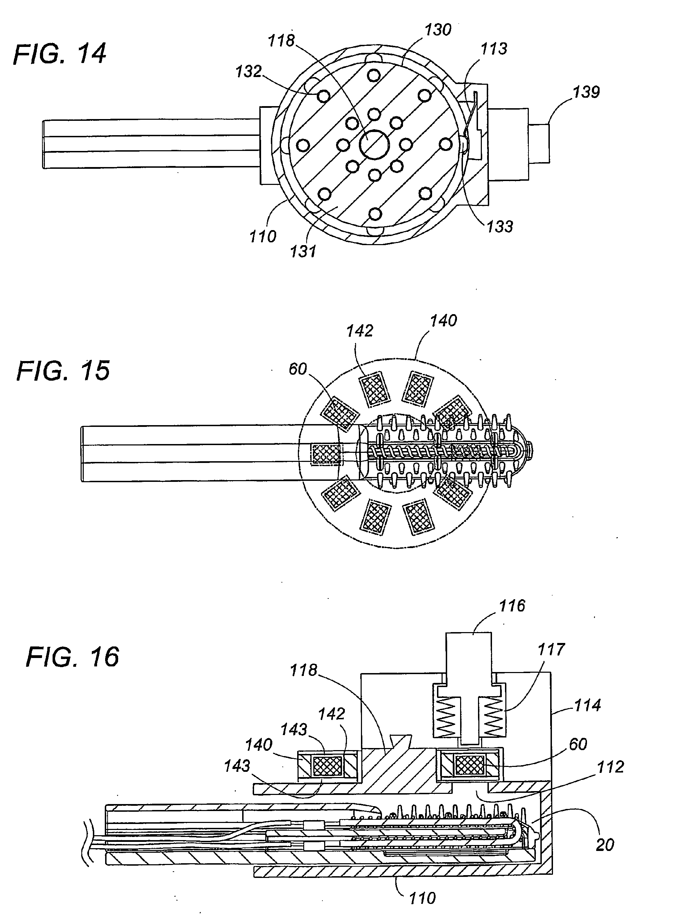

The jacket **134** is formed on its top with handle **135** for rotating the disc **130.** As shown in FIG. 14, a catch spring **113** is provided on the side of the sheath for selective latching engagement with any one of latch projections **133** on the periphery of the disc **130,** giving a clicking rotary motion to the disc **130.** The jacket **134** may be detachable to the base **131** for allowing the refilling of the mascara pieces.

Although the illustrated embodiment discloses that the two compartments come simultaneously into registration with the opening at one time, it is equally possible to arrange the compartments such that a single compartment or more than two compartments come into registration with the opening.

### Third Embodiment <FIGS. 15 and 16>

FIGS. 15 and 16 illustrates a third embodiment of the loading mechanism which is similar to the second embodiment except that the disc **140** is alone provided as a replacement part detachable to the sheath **110** and each compartment **142** is sealed at its upper and lower ends with breakable seals **143** to retain the mascara pieces **60** in the compartment. The disc **140** is detachably mounted around a spindle **118** projecting on the periphery of the sheath **110** to be rotatable about an axis perpendicular to the axis of the sheath **110.** The compartments **142** are circumferentially spaced and are each sealed at its upper and lower open ends respectively by the breakable seals **143** in the form of aluminum foils, for example, to retain therein the mascara piece **60.** The sheath **110** is additionally formed with a detachable base **114** which carries a plunger **116** movable in a direction perpendicular to the axis of the sheath **110.** When the plunger **116** is pressed against the bias of a spring **117,** it breaks the seal **143** and forces the mascara piece **60** out of the compartment **142,** thereby allowing the mascara piece **60** to drop onto the applicator through the opening **112** of the sheath **110**. When the disc **140** is exhausted of the mascara piece **60,** it is replaced by a fresh disc filled with the mascara pieces.

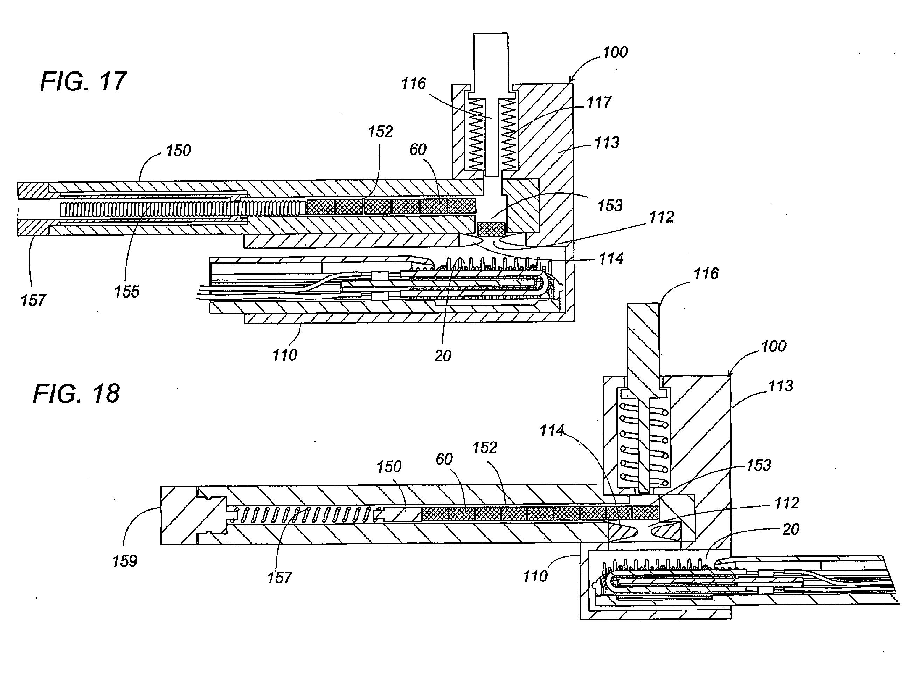

### Fourth Embodiment <FIG. 17>

FIG. 17 illustrates a fourth embodiment of the loading mechanism provided on the side of the cap **100.** The cap **100** includes a sheath **110** configured to accommodate the device, and a holder in the form of a tube **150** which runs parallel to the sheath along the length of the applicator to define therein an elongated compartment **152** for receiving therein a series of mascara pieces **60.** An outlet **153** is formed in the periphery of the tube **150** adjacent to one axial end thereof to deliver the mascara piece **60** onto the applicator **20** through an opening **112** formed in the sheath **110.** A screw-in-piston **155** is inserted in the tube **150** to advance the mascara pieces **60** towards the outlet **153.** The tube **150** is provided at its axial end remote from the outlet **153** with a dial **157** of which rotational motion is translated into a linear motion of the screw-in-piston **155** within the tube **150.** Thus, rotating the dial **157** in one direction advances the piston **155** and therefore the mascara pieces **60** towards the outlet **153.** The mascara piece **60** reaching the outlet **153** is caused to drop into the opening **112** and is retained thereat by means of normally-closed flap valves **114**. A plunger **116** is provided to force the mascara piece **60** out of the opening **112** by temporarily opening the valves **113,** allowing the mascara piece **60** to drop onto the applicator **20.** The plunger **116** is supported to an extension **113** of the sheath to be movable in a direction perpendicular to the axis of the tube **150**. When pressed against the bias of a spring **117,** the plunger **116** has its lower end projected into the tube **150** and into the outlet **153,** forcing the mascara piece **60** out of the opening **112.** Refilling of the mascara pieces is made by removing the dial **157** and the piston **155** out of the tube **150.** The tube **150** may be detachable to the sheath **110** as a replacement part containing a plurality of mascara pieces **60.**

FIG. 18 illustrates a modification of the above embodiment which is identical to the fourth embodiment except for the use of a spring-biased piston 155 in place of the screw-in-piston. The piston **156** is disposed within the tube **150** together with a spring **157** which biases the piston **155** to feed the mascara pieces **60** towards the outlet **153.** An end cap **159** is held in a threaded engagement with one end of the tube **150** to bear the spring **157.** Like parts are designated by like reference numerals for easy reference purpose.

### Fifth Embodiment <FIG. 19>

FIG. 19 illustrates a fifth embodiment of the loading mechanism provided on the side of the cap **100.** The cap **100** includes a sheath **110** configured to receive the device and to have an elongated compartment **162** extending in parallel with an axis of the sheath **110.** The compartment **162** accommodates therein a screw-conveyer **164** which feeds the mascara pieces **60** in the form of pellets supplied at a hopper **166** towards an opening **112** formed at one axial end of the sheath **110.** The opening **112** is disposed adjacent to the applicator **20** for delivering the mascara pieces onto the applicator **20.** The screw-converter **164** is composed of an axle **165** with a spiral fin and is rotatably supported to the sheath **110.** A dial **169** is coupled to the axle **165** for rotating the conveyor **164** and therefore feeding the mascara pieces **60** to the applicator **20** out through the opening **112.**

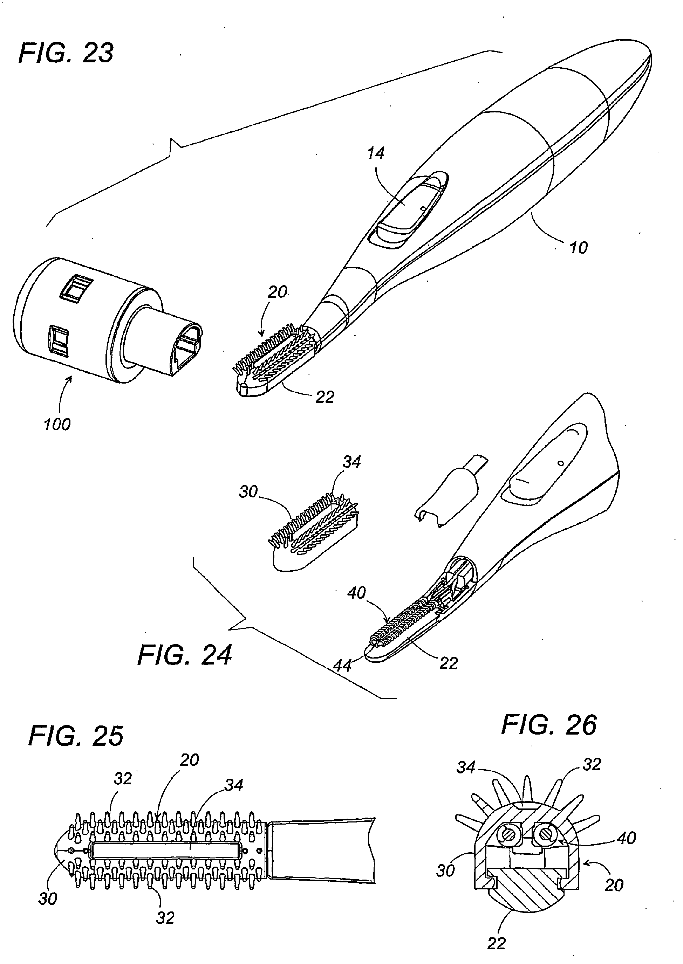

FIGS. 23 to 24 illustrate an eyelash treatment device which may be equally utilized in combination with various mascara feeding caps as discussed hereinbefore. Like parts are designated by like reference numerals for easy reference purpose. The device has a grip **10** and an applicator **20,** which is composed of a head **22** extending integrally from the grip **10,** and a comb attachment **30** detachably fitted on the head **22.** The head **22** is made of a dielectric plastic material and carries the heater **40** composed of a resistor coil **42** wound around a U-shaped core **44** of dielectric material to give two parallel horizontal rows running in the length of the applicator **20.** The coil **42** is electrically connected to a voltage source, i.e., a battery **12** within the grip **10** and is energized by manipulating a switch handle **14** on the side of the grip **10**. The comb attachment **30** is made of a dielectric plastic material and is fitted on the head **22** in thermal contact with the heater **40** so as to be heated at an elevated temperature for softening the mascara composition. The comb attachment **30** is shaped to have a rounded top face provided with a comb having a plurality of comb teeth **32** which are arranged along the length and width of the applicator for applying the softened mascara composition to the eyelashes while smoothening the eyelashes. A ditch **34** is formed in the width center of the top face to extend the length of the applicator **20** for receiving the mascara piece **60** and retaining the softened mascara composition, which is thereafter allowed to flow over the comb teeth. The softened mascara composition climbs-up to the comb teeth **32** by the action of a surface tension to be ready for being delivered to the eyelashes as the comb teeth **32** smoothen the eyelashes.

All documents cited in the Detailed Description of the Invention are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An eyelash treatment device comprising:
a grip (10) to be grasped by a user's hand;
an applicator (20) configured to be supported by said grip and to receive a mascara composition for delivering said mascara composition to eyelashes of the user; and
a cap (100) configured to fit over said applicator and hold said mascara composition,
**characterized in that**
said cap is provided with a loading means (120; 130; 140; 150, 155, 157; 162, 164, 166, 169) which is configured to hold a plurality of pieces (60) of said mascara composition and to load the pieces one by one to said applicator (20).

2. The device according to claim 1, wherein said applicator (20) is elongated to have a length and carries a heater (40) extending along said length for softening said mascara composition by heat and a comb (30), being arranged along the length of said applicator to be coated with said softened mascara composition for delivering to the eye lashes.

3. The device as set forth in claim 1 or 2, wherein
said cap includes a sheath (110) fitted around the applicator and being formed with an opening (112) communicating with said applicator;
said loading means including a carrier (120) which is formed with a plurality of compartments (122) each storing said piece of said mascara composition,
said carrier being movable relative to said sheath to make one of the compartments selectively in registration with said opening for loading the piece of said mascara composition to said applicator through said opening.

4. The device as set forth in claim 3, wherein
said sheath is configured to be rotatively fixed relative to said applicator, and to have an axis extending along a length of said applicator;
said carrier being in the form of a sleeve coaxial with said sheath and having said compartments arranged circumferentially about said axis, and said sleeve being rotatable about said axis relative to said sheath.

5. The device as set forth in claim 4, wherein
said sleeve includes a plurality of gates (127) respectively closing said compartments, each of said gates being movable to open each associated one of said compartments for loading the piece (60) of said mascara composition into said applicator.

6. The device as set forth in claim 4, wherein
said opening is provided with a normally-closed flap valve (114),
said loading means including a plunger (116) which forces the piece of said mascara composition to temporarily open said flap valve for loading the same into said applicator.

7. The deviceas set forth in claim 3, wherein
said sheath is configured to be rotatively fixed relative to said applicator and to have an axis extending along a length of said applicator;
said carrier being in the form of a disc (130; 140) rotatable relative to said sheath about an upright axis perpendicular to the axis of said sheath, and
said disc having the plurality of said compartments (132; 142) circumferentially arranged about said upright axis.

8. The device as set forth in claim 7 , wherein
said loading means includes a gate (137) which normally closes the opening of said sheath,
said gate being movable to an open position for loading the piece of said mascara composition from said compartment into said applicator.

9. The device as set forth in claim 7 , wherein
each piece (60) of said mascara composition is retained in each of said compartments by means of a breakable seal (143),
said loading means including a plunger (116) which forces the piece (60) of said mascara composition out of said breakable seal so as to load the same to said applicator.

10. The device as set forth in claim 7, wherein
said opening is elongated along the axis of the sheath,
said disc (130) being formed with a plurality of said compartments (132) which are spaced circumferentially and radially such that a set of radially aligned compartments comes into registration with said axially arranged openings (112).

11. The device as set forth in claim 1 or 2, wherein
said cap comprises a sheath (110) fitted around the applicator and being formed with a compartment (152) which extends along a length of said applicator for receiving a plurality of pieces of said mascara compositions (60) in series,
said sheath being formed with an opening (112) through which one length end of said compartment communicates with said applicator,
said opening being provided with a normally-closed flap valve (114),
said loading means including a feeder in the form of a screw-in piston (155) that drives the pieces of said mascara composition linearly within said compartment towards said opening,
said loading means further comprising a plunger (116) that forces the piece around said opening against said flap valve to temporarily open the same, thereby loading the piece of said mascara composition into said applicator through said opening.

12. The device as set forth in claim 1 or 2, wherein
said cap comprises a sheath (110) fitted around the applicator and being formed with a compartment (152) which extends along a length of said applicator for receiving a plurality of pieces of said mascara compositions in series,
said compartment being formed at its one lengthwise end with an opening (112) which communicates with said applicator, said opening being provided with a normally-closed flap valve,
said loading means including a feeder in the form of a spring-biased piston (156) that drives the pieces of said mascara composition linearly within said compartment towards said opening,
said loading means further including a plunger (116) which forces the piece around said opening against said flap valve to temporarily open the same, thereby loading the piece of said mascara composition into said applicator through said opening.

13. The device as set forth in claim 1 or 2, wherein
said cap comprises a sheath (110) fitted around the applicator and being formed with a compartment (162) extending along a length of said applicator,
said sheath being formed at its one lengthwise end with an opening (112) through which said compartment communicates with the applicator,
said loading means including a hopper (166) which is formed at the other lengthwise end of said sheath to receive said pieces in the form of pellets,
said loading means including a screw conveyor 164) that extends into said compartment so as to feed the pellets from said hopper to said opening for loading the same into said applicator.

14. A cosmetic product, comprising
an eyelash treatment device according to one of the preceding claims, the cosmetic product further comprising
a mascara composition comprising at least a solid hydrophobic component; said mascara composition having:
i) a needle penetration, as measured according to the ASTM Test Method D5, of from about 1 to about 40 at 25°C;
ii) a yield stress of at least about 1500 Pa at 25°C;
iii) a viscosity of between about 1 mPas and about 10,000,000 mPas at 100°C.

## Patentansprüche

1. Augenwimperbehandlungseinheit umfassend:
einen Griff (10) zum Ergreifen durch eine Hand eines Nutzers;
einen Applikator (20), der ausgebildet ist, um durch den Griff gehalten zu werden und
um Wimperntusche zum Aufbringen auf die Augenwimpern eines Nutzers aufzunehmen; und
eine Kappe (100), die ausgebildet ist, um über den Applikator zu passen und um die Wimperntusche zu halten,
**dadurch gekennzeichnet, dass**
die Kappe mit einem Beladungsmittel (120; 130; 140; 150, 155, 157; 162, 164, 166, 169) bereitgestellt ist, wobei das Beladungsmittel ausgebildet ist, um eine Vielzahl von Bruchstücken (60) der Wimperntusche zu halten und um die Bruchstücke eines nach dem anderen auf den Applikator (20) zu bringen.

2. Einheit nach Anspruch 1, wobei der Applikator (20) sich entlang einer Länge erstreckt und einen Heizer (40) und einen Kamm (30) aufweist, wobei der Heizer (40) sich entlang der Länge erstreckt, um die Wimperntusche durch Hitze aufzuweichen, und der Kamm (30) entlang der Länge des Applikators ausgebildet ist, um sich mit der aufgeweichten Wimperntusche zum Aufbringen auf die Augenwimpern zu bedecken.

3. Einheit nach einem der Ansprüche 1 oder 2, wobei
die Kappe eine Umhüllung (110) umfasst, wobei in die Umhüllung der Applikator eingepasst ist und mit einer Öffnung (112) ausgebildet ist, um mit dem Applikator in Verbindung zu stehen;
wobei das Beladungsmittel einen Träger (120) umfasst, der mit einer Vielzahl von Fächern (122) gebildet ist, wobei jedes der Fächer ein Bruchstück der Wimperntusche aufbewahrt,
wobei der Träger relativ zu der Umhüllung bewegbar ist, um eines der Fächer selektiv in Erfassung durch die Öffnung zu bringen, um den Applikator mit dem Bruchstück der Wimperntusche durch die Öffnung zu beladen.

4. Einheit nach Anspruch 3, wobei
die Umhüllung ausgebildet ist, um relativ zu dem Applikator drehfest zu sein und um eine Achse, die sich entlang der Länge des Applikators erstreckt, zu haben; wobei der Träger in Form einer koaxialen Hülse mit der Umhüllung ausgebildet ist und die Fächer peripher um die Achse herum angeordnet sind und die Hülse um die Achse relativ zu der Umhüllung drehbar ist.

5. Einheit nach Anspruch 4, wobei
die Hülse eine Vielzahl von Blenden (127), die die Fächer entsprechend verschließen, umfasst, wobei jede der Blenden bewegbar ist, um das jeweils entsprechende Fach zum Aufbringen des Bruchstückes (60) der Wimperntusche auf den Applikator zu öffnen.

6. Einheit nach Anspruch 4, wobei
die Öffnung mit einer normal-geschlossenen Ventilklappe (114) ausgebildet ist, wobei das Beladungsmittel einen Kolben (116) umfasst, welcher erzwingt, dass das Bruchstück der Wimperntusche die Ventilklappe öffnet, um den Applikator mit dem Bruchstück zu beladen.

7. Einheit nach Anspruch 3, wobei
die Umhüllung ausgebildet ist, um drehfest relativ zu dem Applikator zu sein und um eine Achse, die sich entlang einer Länge des Applikators erstreckt, zu haben; wobei der Träger in Form einer Scheibe (130; 140), die relativ zu der Umhüllung um eine aufrechte Achse senkrecht zu der Achse der Umhüllung drehbar ist, ausgebildet ist, und
die Scheibe eine Vielzahl von den Fächern (132; 142), die sich entlang des Umfanges um die aufrechte Achse erstrecken, hat.

8. Einheit nach Anspruch 7, wobei
das Beladungsmittel eine Blende umfasst, welche normalerweise die Öffnung der Umhüllung schließt,
wobei die Blende in eine offene Position bewegbar ist, um das Bruchstück der Wimperntusche von dem Fach auf den Applikator zu beladen.

9. Einheit nach Anspruch 7, wobei
jedes Bruchstück (60) der Wimperntusche in jedem der Fächer durch ein zerbrechliches Siegel (143) gehalten wird,
wobei das Beladungsmittel einen Kolben (116) umfasst, welcher das Bruchstück (60) der Wimperntusche aus dem zerbrechlichen Siegel heraus zwingt, um den Applikator mit dem Bruchstück zu beladen.

10. Einheit nach Anspruch 7, wobei
die Öffnung sich entlang der Achse der Umhüllung erstreckt,
die Scheibe (130) mit einer Vielzahl von Fächern (132) gebildet ist, welche entlang des Umfanges voneinander getrennt angeordnet sind und radial derart voneinander getrennt sind, dass ein Satz radial ausgerichteter Fächer mit den axial angeordneten Öffnungen (112) in Verbindung tritt.

11. Einheit nach Anspruch 1 oder 2, wobei
die Kappe eine Umhüllung (110) aufweist, wobei die Umhüllung um den Applikator passt und mit einem Fach (152) ausgebildet ist, welches sich entlang einer Länge des Applikators erstreckt, um eine Vielzahl von Bruchstücken von Wimperntusche (60) reihenweise aufzunehmen,
wobei die Umhüllung mit einer Öffnung (112) ausgebildet ist, durch welche ein Längenende des Faches mit dem Applikator kommuniziert,
die Öffnung mit einer normal-verschlossenen Ventilklappe (114) ausgebildet ist, das Beladungsmittel einen Beschicker in Form eines hineindrehbaren Schafts (155) umfasst, welcher die Bruchstücke der Wimperntusche linear in die Fächer hin zu der Öffnung bewegt,
das Beladungsmittel ferner einen Kolben (116) umfasst, der das Bruchstück an der Öffnung gegen die Ventilklappe drückt, um zeitweise dieselbe zu öffnen, wodurch das Bruchstück der Wimperntusche auf den Applikator durch die Öffnung befördert wird.

12. Einheit nach Anspruch 1 oder Anspruch 2, wobei
die Kappe eine Umhüllung (110) aufweist, wobei in die Umhüllung der Applikator eingepasst ist und mit einem Fach (152) ausgebildet ist, welches sich entlang der Länge des Applikators erstreckt, um eine Vielzahl von Bruchstücken der Wimperntusche reihenweise aufzunehmen,
das Fach mit einer Öffnung (112) entlang einer seiner längsseitigen Enden ausgebildet ist, wobei die Öffnung (112) mit dem Applikator in Verbindung steht, wobei die Öffnung mit einer normal-geschlossenen Ventilklappe ausgebildet ist,
das Beladungsmittel einen Beschicker in Form eines Feder-vorgespannten Schafts (156) umfasst, der die Bruchstücke der Wimperntusche linear innerhalb des Faches zur Öffnung hin bewegt,
das Beladungsmittel ferner einen Kolben (116) umfasst, welcher das Bruchstück in der Nähe der Öffnung zwingt, die Ventilklappe zeitweise zu öffnen, wodurch der Applikator mit dem Bruchstück der Wimperntusche durch die Öffnung beladen wird.

13. Einheit nach Anspruch 1 oder Anspruch 2, wobei
die Kappe eine Umhüllung (110) aufweist, wobei in die Umhüllung der Applikator eingepasst ist und mit einem Fach (162) ausgebildet ist, welches sich entlang der Länge des Applikators erstreckt,
die Umhüllung an einem seiner längsseitigen Enden mit einer Öffnung (112) ausgebildet ist, durch welche das Fach mit dem Applikator in Verbindung steht,
das Beladungsmittel einen Dosiertrichter (166) umfasst, welcher an dem anderen längsseitigen Ende von der Umhüllung ausgebildet ist, um die Bruchstücke in Form von Pellets aufzunehmen,
das Beladungsmittel einen Schneckenförderer (164) umfasst, welcher sich in das Fach hinein erstreckt, so dass die Pellets von dem Dosiertrichter zu der Öffnung zum Beladen des Applikators transportiert werden.

14. Ein kosmetisches Produkt, umfassend:
eine Augenwimperbehandlungseinheit entsprechend einem der vorhergehenden Ansprüche, wobei das kosmetische Produkt weiter umfasst:
eine Wimperntusche, die zumindest eine feste hydrophobishe Komponente aufweist,
wobei die Wimperntusche:
i) eine Nadelpenetration, die entsprechend der ASTM Testmethode D5 mit einem Wert von ungefähr 1 bis zu ungefähr 40 bei 25° C gemessen wurde;
ii) eine Fließspannung von zumindest 1500 Pa bei 25° C;
iii) eine Viskosität zwischen ungefähr 1 mPas und ungefähr 10,000,000 mPas bei 100° C aufweist.

## Revendications

1. Dispositif de traitement de cils, comportant :
une poignée (10) devant être saisie par une main d'utilisateur,
un applicateur (20) configuré pour être supporté par ladite poignée et pour recevoir une composition de mascara pour distribuer ladite composition de mascara sur les cils de l'utilisateur, et
un capuchon (100) configuré pour s'insérer au-dessus dudit applicateur et contenir ladite composition de mascara,
**caractérisé en ce que**
ledit capuchon est muni de moyens de chargement (120 ; 130 ; 140 ; 150, 155, 157 ; 162, 164, 166, 169) qui sont configurés pour contenir une pluralité de fragments (60) de ladite composition de mascara et pour charger les fragments un à un dans ledit applicateur (20).

2. Dispositif selon la revendication 1, dans lequel ledit applicateur (20) est allongé pour avoir une longueur et porte un dispositif de chauffage (40) s'étendant le long de ladite longueur pour ramollir ladite composition de mascara par de la chaleur et un peigne (30), agencé le long de la longueur dudit applicateur pour être revêtu de ladite composition de mascara ramollie pour une distribution sur les cils.

3. Dispositif tel qu'exposé dans la revendication 1 ou 2, dans lequel
ledit capuchon inclut une gaine (110) agencée autour de l'applicateur et étant formée munie d'une ouverture (112) communiquant avec ledit applicateur,
lesdits moyens de chargement incluant un support (120) qui est formé muni d'une pluralité de compartiments (122) contenant chacun ledit fragment de ladite composition de mascara,
ledit support étant mobile par rapport à ladite gaine pour faire concorder sélectivement l'un des compartiments avec ladite ouverture pour charger le fragment de ladite composition de mascara dans ledit applicateur à travers ladite ouverture.

4. Dispositif tel qu'exposé dans la revendication 3, dans lequel
ladite gaine est configurée pour être fixe en rotation par rapport audit applicateur, et pour avoir un axe s'étendant le long d'une longueur dudit applicateur,
ledit support se présentant sous la forme d'un manchon coaxial à ladite gaine et ayant lesdits compartiments agencés de manière circonférentielle autour dudit axe, et ledit manchon pouvant tourner autour dudit axe par rapport à ladite gaine.

5. Dispositif tel qu'exposé dans la revendication 4, dans lequel
ledit manchon inclut une pluralité de portillons (127) fermant respectivement lesdits compartiments, chacun desdits portillons étant mobile pour ouvrir chaque compartiment associé desdits compartiments pour charger le fragment (60) de ladite composition de mascara dans ledit applicateur.

6. Dispositif tel qu'exposé dans la revendication 4, dans lequel
ladite ouverture est munie d'une soupape à clapet normalement fermée (114),
lesdits moyens de changement incluant un poussoir (116) qui force le fragment de ladite composition de mascara à ouvrir temporairement ladite soupape à clapet pour charger celui-ci dans ledit applicateur.

7. Dispositif tel qu'exposé dans la revendication 3, dans lequel
ladite gaine est configurée pour être fixe en rotation par rapport audit applicateur et pour avoir un axe s'étendant le long d'une longueur dudit applicateur,
ledit support se présentant sous la forme d'un disque (130 ; 140) pouvant tourner par rapport à ladite gaine autour d'un axe vertical perpendiculaire à l'axe de ladite gaine, et
ledit disque ayant la pluralité desdits compartiments (132 ; 142) agencés de manière circonférentielle autour dudit axe vertical.

8. Dispositif tel qu'exposé dans la revendication 7, dans lequel
lesdits moyens de chargement incluent un portillon (137) qui obture normalement l'ouverture de ladite gaine,
ledit portillon pouvant être amené à une position ouverte pour charger le fragment de ladite composition de mascara à partir du compartiment dans ledit applicateur.

9. Dispositif tel qu'exposé dans la revendication 7, dans lequel
chaque fragment (60) de ladite composition de mascara est retenu dans chacun desdits compartiments au moyen d'un joint cassable (143),
lesdits moyens de chargement incluant un poussoir (116) qui force le fragment (60) de ladite composition de mascara à l'extérieur dudit joint cassable de manière à charger celui-ci dans ledit applicateur.

10. Dispositif tel qu'exposé dans la revendication 7, dans lequel
ladite ouverture est allongée le long de l'axe de la gaine,
ledit disque (130) étant formé muni d'une pluralité desdits compartiments (132) qui sont espacés de manière circonférentielle et radiale de telle sorte qu'un ensemble de compartiments radialement alignés vient en concordance avec lesdites ouvertures agencées axialement (112).

11. Dispositif tel qu'exposé dans la revendication 1 ou 2, dans lequel
ledit capuchon comporte une gaine (110) agencée autour de l'applicateur et étant formée munie d'un compartiment (152) qui s'étend le long d'une longueur dudit applicateur pour recevoir une pluralité de fragments de ladite composition de mascara (60) en série,
ladite gaine étant formée munie d'une ouverture (112) par l'intermédiaire de laquelle une extrémité longitudinale dudit compartiment communique avec ledit applicateur,
ladite ouverture étant munie d'une soupape à clapet normalement fermée (114),
lesdits moyens de chargement incluant un dispositif d'alimentation ayant la forme d'un piston à vis (155) qui entraîne linéairement les fragments de ladite composition de mascara à l'intérieur dudit compartiment vers ladite ouverture,
lesdits moyens de chargement comportant en outre un poussoir (116) qui force le fragment autour de ladite ouverture contre ladite soupape à clapet pour ouvrir temporairement celle-ci, en chargeant ainsi le fragment de ladite composition de mascara dans ledit applicateur à travers ladite ouverture.

12. Dispositif tel qu'exposé dans la revendication 1 ou 2, dans lequel
ledit capuchon comporte une gaine (110) agencée autour de l'applicateur et étant formée munie d'un compartiment (152) qui s'étend le long d'une longueur dudit applicateur pour recevoir une pluralité de fragments desdites compositions de mascara en série,
ledit compartiment étant formé, au niveau de son extrémité longitudinale, muni d'une ouverture (112) qui communique avec ledit applicateur, ladite ouverture étant munie d'une soupape à clapet normalement fermée,
lesdits moyens de chargement incluant un dispositif d'alimentation ayant la forme d'un piston rappelé par ressort (156) qui entraîne linéairement les fragments de ladite composition de mascara à l'intérieur dudit compartiment vers ladite ouverture,
lesdits moyens de chargement incluant en outre un poussoir (116) qui force le fragment autour de ladite ouverture contre la soupape à clapet pour ouvrir temporairement celle-ci, en chargeant ainsi le fragment de ladite composition de mascara dans ledit applicateur à travers ladite ouverture.

13. Dispositif tel qu'exposé dans la revendication 1 ou 2, dans lequel
ledit capuchon comporte une gaine (110) agencée autour de l'applicateur et étant formée munie d'un compartiment (162) s'étendant le long d'une longueur dudit applicateur,
ladite gaine étant formée, au niveau de son extrémité longitudinale, munie d'une ouverture (112) par l'intermédiaire de laquelle ledit compartiment communique avec l'applicateur,
lesdits moyens de chargement incluant une trémie (166) qui est formée sur l'autre extrémité longitudinale de ladite gaine pour recevoir lesdits fragments sous la forme de granules,
lesdits moyens de chargement incluant un convoyeur à vis (164) qui s'étend dans ledit compartiment de manière à acheminer les granules de ladite trémie à ladite ouverture pour charger ceux-ci dans ledit applicateur.

14. Produit cosmétique, comportant :
un dispositif de traitement de cils selon l'une des revendications précédentes, le produit cosmétique comportant en outre :
une composition de mascara comportant au moins un composant hydrophobe solide, ladite composition de mascara ayant :
i) une pénétration à l'aiguille, lorsque mesurée selon la méthode d'essai ASTM D5, d'environ 1 à environ 40 à 25°C,
ii) une contrainte à la limite élastique d'au moins environ 1 500 Pa à 25°C,
iii) une viscosité comprise entre environ 1 mPas et environ 10 000 000 mPas à 100°C.
